# EUROPEAN PATENT APPLICATION

(11) **EP 2 674 157 A1**
(43) Date of publication of application: **18.12.2013**
(21) Application number: 12744302.6
(22) Date of filing: 06.02.2012
(51) Int. Cl.: A61K 31/202, A61K 31/232, A61K 45/00, A61P 9/04

(54) **THERAPEUTIC AGENT FOR DIASTOLIC CONGESTIVE HEART FAILURE**

(30) Priority: 07.02.2011 JP 2011023542; 24.03.2011 JP 2011066460
(71) Applicant: Mochida Pharmaceutical Co., Ltd., Tokyo 160-8515 (JP)
(72) Inventor: OHNISHI, Haruo, Tokyo 160-8515 (JP)
(74) Representative: Keirstead, Tanis Evelyne
(86) International application number: PCT/JP2012/052606
(87) International publication number: WO 2012/108379

(57) **Abstract**

The present invention provides a pharmaceutical composition for the treatment of diastolic congestive heart failure containing at least one active ingredient selected from the group consisting of icosapentaenoic acid, a pharmaceutically acceptable salt thereof, and an ester thereof.

## Description

### TECHNICAL FIELD

The present invention relates to a pharmaceutical composition for the treatment of diastolic congestive heart failure and a method for treating diastolic congestive heart failure.

### BACKGROUND ART

Heart failure occurs when the heart is unable to provide sufficient pump functions to supply blood flow required by the tissue metabolism of the body. Congestive heart failure refers to heart failure with pulmonary and/or peripheral congestive symptoms resulting from circulating blood volume increased by reduced cardiac output.

The congestive heart failure includes systolic congestive heart failure with a poor left ventricular systolic function (left ventricular ejection fraction of 40% or lower) and diastolic congestive heart failure that exhibits apparent symptoms of congestive heart failure, but maintains a left ventricular systolic function (left ventricular ejection fraction larger than 40%, for example, 45% to 50% or higher) (Non Patent Literature 1). The concept of diastolic congestive heart failure (or diastolic heart failure), which should be definitively distinguished from systolic congestive heart failure, was proposed in 1996 for the first time (Non Patent Literature 2). According to Guidelines for Treatment of Chronic Heart Failure (JCS 2010), p. 4, (Japan), heart failure with reduced left ventricular contractility is classified into "systolic failure", while heart failure in which left ventricular contractility is kept is classified into "diastolic failure" in the diagnosis of chronic heart failure. The diastolic congestive heart failure is synonymous with this "diastolic failure". Congestion is a main symptom of chronic heart failure. The congestive heart failure is synonymous with chronic heart failure that exhibits congestive symptoms.

Although some reports state that the prognosis of diastolic congestive heart failure is more favorable than that of systolic congestive heart failure (or systolic heart failure), the diastolic congestive heart failure generally results in a poor prognosis. In this regard, the diastolic congestive heart failure is similar in prognosis to the systolic congestive heart failure (Non Patent Literature 3).

The number of congestive heart failure patients in the USA is 4,600,000 people, 30% to 50% of which have diastolic congestive heart failure (Non Patent Literature 4). Specifically, estimated 1,400,000 to 2,300,000 patients suffer from diastolic congestive heart failure in the USA and are still increasing (Non Patent Literature 5).

In Japan, diastolic congestive heart failure patients reportedly account for 26% (Non Patent Literature 3), 34% (Non Patent Literature 6), or 67.8% (Non Patent Literature 7), etc. of congestive heart failure patients. Approximately 2,500,000 Japanese people (80% or more of which are people aged 65 or over) are affected by congestive heart failure, which is presumed to kill approximately 20,000 people every year (Non Patent Literature 8). The number of diastolic congestive heart failure patients is estimated at approximately 650,000 to 1,700,000 people. Since elderly people account for the high percentage of diastolic congestive heart failure patients, the number of diastolic congestive heart failure patients will increase in Japan's aging society.

Most of large clinical trials on the treatment of heart failure are directed to systolic failure cases. The treatment of diastolic failure has not been sufficiently evaluated. Treatment strategy for diastolic failure has not yet been established (Non Patent Literature 9). In fact, the report of Mayo Clinic which observed heart failure patients under common treatment from 1987 through 2001 shows that although the survival rate of systolic congestive heart failure was improved in observation from 1997 to 2001 compared with observation from 1987 to 1991, no such improvement was seen in diastolic congestive heart failure (Non Patent Literature 5). Thus, treatment strategy for diastolic congestive heart failure should be urgently established.

Possible pathological conditions of diastolic failure are basically (1) increased ventricular stiffness, (2) impaired relaxation, (3) diastolic ventricular failure caused by epicardial thickening, and (4) diastolic left ventricular failure caused by right ventricular load. Examples of causes of the increased ventricular stiffness include myocardial ischemia, cardiomyocyte hypertrophy caused mainly by the mechanical stimulation of cardiac muscle, and myocardial fibrosis induced by liquid factors such as cytokines. Also, the ventricular stiffness is increased by aging.

The relation of diastolic dysfunction and ventricular stiffness to epicardial fat has received attention.

Reportedly, increase in the thickness of epicardial fat is related to change in left ventricular weight (Non Patent Literature 10) and change in diastolic function (Non Patent Literature 11) and also correlates with atrial enlargement and impairment in the diastolic filling of the right and left ventricles (Non Patent Literature 12). It has been further reported that: although significant increase in left ventricular weight and diastolic dysfunction were observed in hypertension patients having 7 mm or thicker epicardial fat compared with patients having 7 mm or thinner epicardial fat, these phenomena were not confirmed in patients in the same cohort classified depending on the presence or absence of excess abdominal visceral fat; and the significantly increased left ventricular weight or the diastolic dysfunction was shown to be related to epicardial fat but not related to abdominal visceral fat (Non Patent Literature 13). Also, the amount of epicardial fat reportedly decreases in systolic heart failure associated with ischemic cardiomyopathy and dilated cardiomyopathy (Non Patent Literature 14).

There is a report showing the involvement of angiotensin II in myocardial fibrosis (Non Patent Literature 15). In the presence of angiotensin II, cardiomyocytes cause the production of TGF-β, which in turn induces IL-6 from fibroblasts to promote collagen synthesis (Non Patent Literature 16). The expression of angiotensinogen mRNA in epicardial fat was reportedly at the same level as that in substernal mediastinal fat, but was 5.5 times the expression in abdominal subcutaneous fat and 1.9 times the expression in omental fat tissue (Non Patent Literature 17).

Cardiomegaly or cardiac hypertrophy is the compensatory response of cardiac muscle tissue to increase in mechanical load. The hypertrophic response occurs subsequently to stretch, a mechanical factor. Angiotensin II, endothelin-1, and TGF-β are known to act as mediators of cardiomyocyte hypertrophy induced by the stretch (Non Patent Literature 18). The expression of endothelin-1 mRNA in epicardial fat, as in the expression of angiotensinogen mRNA, was reportedly at the same level as that in substernal mediastinal fat, but was 1.6 times the expression in abdominal subcutaneous fat (Non Patent Literature 17).

The involvement of, for example, IL-6, in fibrosis is further known. Left ventricular hypertrophy, increased ventricular stiffness, cardiomyocyte hypertrophy, and elevated collagen levels were observed in rats that received the injection of IL-6 (Non Patent Literature 19).

In some cases, heart failure patients who maintained a systolic function were confirmed to have both stiff ventricle and vascular sclerosis (Non Patent Literature 20). A pulse wave velocity, which reflects vascular stiffness, was 1804 cm/s in diastolic heart failure patients (E/A ratio < 0.75) and was faster than 1573 cm/s in patients without diastolic heart failure (E/A ratio > 0.75) (Non Patent Literature 21), indicating that the pulse wave velocity serving as an index for vascular stiffness was increased in diastolic heart failure with increased cardiac muscular stiffness.

It has been reported that: the amount of epicardial fat in atrial fibrillation patients and persistent atrial fibrillation patients was increased compared with a control (Non Patent Literature 22); and atrial fibrillation inducibility in some cases disappeared by the removal of epicardial fat (Non Patent Literature 23).

Inflammatory cytokines and adipocytokines produced by epicardial fat cells act directly on cardiomyocytes positioned close to the fat cells, probably resulting in atrial fibrillation (Non Patent Literature 24). It has been reported that: the mRNA expression profiles of inflammatory cytokines and adipocytokines in epicardial fat tissue differ from those in abdominal subcutaneous fat tissue, omental fat tissue, and thigh subcutaneous fat tissue (Non Patent Literatures 17 and 25); and atrial fibrillation inducibility is not related to abdominal visceral fat or subcutaneous fat while epicardial fat tissue is more highly related to the development of atrial fibrillation than visceral fat (Non Patent Literature 24).

Study on the effectiveness of an ω3 polyunsaturated fatty acid on cardiac disease has also been reported, but has fallen within the scope of improvement in systolic function.

Duda MK et al. have prepared heart failure rats characterized by systolic dysfunction and left ventricular remodeling by the ligation of the aorta and reported that these phenomena were attenuated by the administration of fish oil condensates (Ocean Nutrition, EPA: 21%, DHA: 49%) (Non Patent Literature 26). Similarly, the administration of tuna fish oil containing 29.3% of saturated fatty acid, 10.8% of an ω6 polyunsaturated fatty acid, and 22.8% of an ω3 polyunsaturated fatty acid to monkeys has been reported to enhance ventricular filling, thus providing increase in left ventricular ejection fraction and a rise in electrical threshold induced by ventricular fibrillation (Non Patent Literature 27). Alternatively, International Publication No. WO2002/058793 (Patent Literature 1) describes use of essential fatty acid containing a mixture of icosapentaenoic acid ethyl ester and docosahexaenoic acid ethyl ester for the treatment of cardiac dysfunction and heart failure attributed to reduced contractility. International Publication No. W02003/068216 (Patent Literature 2) describes the effects of an ω3 polyunsaturated fatty acid on the alleviation of the risk of sudden death of patients affected by heart failure.

The report of the GISSI-HF trial directed to chronic heart failure patients shows that, of 3494 chronic heart failure patients who received concentrated fish oil (1 g/day; which contained 850 to 880 mg of EPA ethyl ester and DHA ethyl ester at a ratio of 1:1.2 in 1 g), 1981 (57%) patients died or were admitted to hospital for cardiovascular reasons and were significantly fewer than 2053 (59.0%) out of 3481 people in the control group (Non Patent Literature 28 and Patent Literature 3). This report also shows that, particularly, in the case of only patients having a left ventricular ejection fraction of 40% or lower, 1788 (56.6%) patients in the concentrated fish oil-administered group (3161 people) died or were admitted to hospital for cardiovascular reasons and were fewer than 1871 (59.2%) out of 3161 people in the control group, whereas 193 (58.0%) patients in the concentrated fish oil-administered group (333 people) of patients having a left ventricular ejection fraction larger than 40% died or were admitted to hospital for cardiovascular reasons and were more than 182 (56.9%) out of 320 people in the control group.

### CITATION LIST

### PATENT LITERATURE

Patent Literature 1: International Publication No. WO2002/058793
Patent Literature 2: International Publication No. WO2003/068216
Patent Literature 3: International Publication No. WO2010/015335

### NON PATENT LITERATURE

Non Patent Literature 1: Vasan RS et al., Circulation. 2000; Vol. 101: pp. 2118
Non Patent Literature 2: Vasan RS et al., Arch Intern Med. 1996; 156: 1789
Non Patent Literature 3: Tsutsui H et al., Cir J. 2006: 70: 1617
Non Patent Literature 4: Vasan RS, Benjamin EJ. N Engl J Med. 2001; 344: 56
Non Patent Literature 5: Owan TE et al., N Engl J Med. 2006; 355: 251
Non Patent Literature 6: Kawashiro N et al., Cir J. 2008; 72: 2015
Non Patent Literature 7: Hiroaki Senba, et al., The 74th Annual Scientific Meeting of the Japanese Circulation Society, 2010
Non Patent Literature 8: Toru Izumi, Proceedings of the 122nd Symposium of the Japanese Association of Medical Sciences, 2002, Frontier of heart failure: treatment and diagnosis, published by the Japanese Association of Medical Sciences, p. 6
Non Patent Literature 9: Guidelines for Treatment of Chronic Heart Failure (JCS 2010); pp. 26-28
Non Patent Literature 10: Iacobellis G, Ribaudo MC et al., Am J Cardiol. 2004; 94: 1084
Non Patent Literature 11: Iacobellis G, Pond CM et al., Obesity (Silver Spring). 2006; 14: 1679
Non Patent Literature 12: Iacobellis G, Leonetti F et al., Int J Cardiol. 2007; 115: 272
Non Patent Literature 13: Natale F et al., Eur J Echocardiology. 2009; 10: 549
Non Patent Literature 14: Doesch C et al., J Cardiovasc Magn Reson. 2010; 12: 40
Non Patent Literature 15: Kuwahara F et al., Hypertension. 2004; 43: 739
Non Patent Literature 16: Sarkar S et al., Am J Physiol Heart Circ Physiol. 2004; 287: H107
Non Patent Literature 17: Fain JN et al., Metabolism. 2010; 59: 1379
Non Patent Literature 18: van Wamel AJ et al., Mol Cell Biochem. 2001; 218: 113
Non Patent Literature 19: Melendez GC et al., Hypertension. 2010; 56: 225
Non Patent Literature 20: Kass DA. Hypertension 2005; 46: 185
Non Patent Literature 21: Yambe M et al., Hypertension Res. 2004; 27: 625
Non Patent Literature 22: M. Obadah Al Chekakie et al., J Am Coll Cardiol. 2010; 56: 784
Non Patent Literature 23: Chang D, Zhang S et al., Circ J. 2010; 74: 885
Non Patent Literature 24: Lin YK, Chen YJ et al., Med Hypotheses. 2010; 74: 1026
Non Patent Literature 25: Baker AR et al., Cardiovasc Diabetol. 2006; 5: 1
Non Patent Literature 26: Duda MK et al., Cardiovascular Research. 2009; 81: 319
Non Patent Literature 27: McLennan PL et al., Cardiovascular Research. 1992; 26: 871
Non Patent Literature 28: Gissi-HF Investigators. Lancet. 2008; 372: 1223

### SUMMARY OF INVENTION

### TECHNICAL PROBLEM

The number of patients with diastolic congestive heart failure is not few and is increasing due to the aging population. Their prognoses are not always favorable. Since diastolic congestive heart failure and systolic congestive heart failure differ in cause and cardiac function, any method for treating systolic congestive heart failure cannot be applied to the treatment of diastolic congestive heart failure. At present, the treatment of diastolic congestive heart failure involves administering renin-angiotensin system inhibitors, diuretics, β blockers, etc., but requires multipronged strategies including improvement in ventricular stiffness, improvement in diastolic function, and alleviation of atrial fibrillation, for obtaining essential therapeutic effects on diastolic congestive heart failure. Particularly, the treatment for improvement in ventricular stiffness has not yet been established in any way. Current treatment methods are still less than sufficient. Meanwhile, the number of elderly people affected by diastolic congestive heart failure will increase in the aging society. Thus, means of treating diastolic congestive heart failure that is capable of improving or preventing, with few adverse reactions, diastolic congestive heart failure-derived symptoms, i.e., edema, dyspnea or shortness of breath, increased ventricular stiffness, cardiac diastolic dysfunction, and/or atrial fibrillation, has been demanded.

### SOLUTION TO PROBLEM

The present inventor has completed the present invention by finding that the continuous administration of an ω3 polyunsaturated fatty acid, particularly, EPA, DHA, or α-linolenic acid, a pharmaceutically acceptable salt thereof, or an ester (e.g., ethyl ester) thereof, or their mixture to a diastolic congestive heart failure patient, particularly, a diastolic congestive heart failure patient having excess epicardial fat can improve or prevent diastolic congestive heart failure-derived symptoms, i.e., edema, dyspnea or shortness of breath, increased ventricular stiffness, cardiac diastolic dysfunction, and/or atrial fibrillation.

Specifically, an aspect of the present invention provides a pharmaceutical composition described in the following (1) to (12):
(1) A pharmaceutical composition for the treatment of diastolic congestive heart failure containing at least one active ingredient selected from the group consisting of an ω3 polyunsaturated fatty acid, a pharmaceutically acceptable salt thereof, and an ester thereof.
(2) The pharmaceutical composition according to (1), wherein the diastolic congestive heart failure is with the deposition of 5 mm or thicker epicardial fat.
(3) The pharmaceutical composition according to (1), wherein the diastolic congestive heart failure is with the deposition of 7 mm or thicker epicardial fat.
(4) The pharmaceutical composition according to any of (1) to (3), wherein the pharmaceutical composition is used for improvement in any one or more diastolic congestive heart failure-derived abnormality (abnormalities) selected from edema, dyspnea or shortness of breath, increased ventricular stiffness, cardiac diastolic dysfunction, and atrial fibrillation.
(5) The pharmaceutical composition according to (4), wherein the diastolic congestive heart failure-derived abnormality is increased ventricular stiffness or cardiac diastolic dysfunction.
(6) A pharmaceutical composition for improvement in cardiac diastolic dysfunction containing at least one active ingredient selected from the group consisting of an ω3 polyunsaturated fatty acid, a pharmaceutically acceptable salt thereof, and an ester thereof, wherein the pharmaceutical composition is used for a congestive heart failure patient who has a left ventricular ejection fraction larger than 40% and maintains a left ventricular systolic function.
(7) The pharmaceutical composition according to (6), wherein the pharmaceutical composition improves at least one index for the cardiac diastolic dysfunction selected from the group consisting of a ratio of a peak filling velocity of early diastolic transmitral flow to a peak filling velocity of atrial systolic transmitral flow (E/A ratio), a deceleration time (DT), an early diastolic mitral annular velocity (E'), and a ratio of the peak filling velocity of early diastolic transmitral flow to the early diastolic mitral annular velocity (E/E' ratio).
(8) The pharmaceutical composition according to any of (1) to (7), wherein the pharmaceutical composition is used for improvement in the prognosis of the diastolic congestive heart failure.
(9) The pharmaceutical composition according to any of (1) to (8), wherein the pharmaceutical composition is used in combination with a drug selected from a renin-angiotensin system inhibitor, a diuretic, a β blocker, and a Ca channel inhibitor.
(10) The pharmaceutical composition according to any of (1) to (9), wherein the ω3 polyunsaturated fatty acid, the pharmaceutically acceptable salt thereof, or the ester thereof is at least one compound selected from the group consisting of icosapentaenoic acid, docosahexaenoic acid, and α-linolenic acid, pharmaceutically acceptable salts thereof, and esters thereof.
(11) The pharmaceutical composition according to (10), wherein the pharmaceutical composition contains icosapentaenoic acid ethyl ester as an active ingredient.
(12) The pharmaceutical composition according to (10), wherein the pharmaceutical composition is administered, for use, at a dose of 1.2 g/day or higher in terms of the total amount of icosapentaenoic acid ethyl ester and docosahexaenoic acid ethyl ester.

An alternative aspect of the present invention provides a pharmaceutical composition described in the following (13) to (23):
(13) A pharmaceutical composition for the treatment of diastolic congestive heart failure containing at least one active ingredient selected from the group consisting of icosapentaenoic acid, a pharmaceutically acceptable salt thereof, and an ester thereof.
(14) The pharmaceutical composition according to (13), the diastolic congestive heart failure is with the deposition of 5 mm or thicker epicardial fat.
(15) The pharmaceutical composition according to (13), wherein the diastolic congestive heart failure is with the deposition of 7 mm or thicker epicardial fat.
(16) The pharmaceutical composition according to any of (13) to (15), wherein the pharmaceutical composition is used for improvement in any one or more diastolic congestive heart failure-derived abnormalities (abnormalities) selected from edema, dyspnea or shortness of breath, increased ventricular stiffness, cardiac diastolic dysfunction, and atrial fibrillation.
(17) The pharmaceutical composition according to (16), wherein the diastolic congestive heart failure-derived abnormality is increased ventricular stiffness or cardiac diastolic dysfunction.
(18) A pharmaceutical composition for improvement in cardiac diastolic dysfunction containing at least one active ingredient selected from the group consisting of icosapentaenoic acid, a pharmaceutically acceptable salt thereof, and an ester thereof, wherein the pharmaceutical composition is used for a congestive heart failure patient who has a left ventricular ejection fraction larger than 40% and maintains a left ventricular systolic function.
(19) The pharmaceutical composition according to (18), wherein the pharmaceutical composition improves at least one index for the cardiac diastolic dysfunction selected from the group consisting of a ratio of a peak filling velocity of early diastolic transmitral flow to a peak filling velocity of atrial systolic transmitral flow (E/A ratio), a deceleration time (DT), an early diastolic mitral annular velocity (E'), and a ratio of the peak filling velocity of early diastolic transmitral flow to the early diastolic mitral annular velocity (E/E' ratio).
(20) The pharmaceutical composition according to any of (13) to (19), wherein the pharmaceutical composition is used for improvement in the prognosis of the diastolic congestive heart failure.
(21) The pharmaceutical composition according to any of (13) to (20), wherein the pharmaceutical composition is used in combination with a drug selected from a renin-angiotensin system inhibitor, a diuretic, a β blocker, and a Ca channel inhibitor.
(22) The pharmaceutical composition according to any of (13) to (21), wherein the pharmaceutical composition contains icosapentaenoic acid ethyl ester as an active ingredient.
(23) The pharmaceutical composition according to (22), wherein the pharmaceutical composition is administered, for use, at a dose of 1.2 g/day or higher in terms of the amount of icosapentaenoic acid ethyl ester.

A further alternative aspect of the present invention provides a method described in the following (24) to (35):
(24) A method for treating diastolic congestive heart failure, comprising administering at least one compound selected from the group consisting of an ω3 polyunsaturated fatty acid, a pharmaceutically acceptable salt thereof, and an ester thereof to a patient.
(25) The method according to (24), wherein the diastolic congestive heart failure is with the deposition of 5 mm or thicker epicardial fat.
(26) The method according to (24), wherein the diastolic congestive heart failure is with the deposition of 7 mm or thicker epicardial fat.
(27) The method according to any of (24) to (26), wherein the method is used for improvement in any one or more diastolic congestive heart failure-derived abnormalities (abnormalities) selected from edema, dyspnea or shortness of breath, increased ventricular stiffness, cardiac diastolic dysfunction, and atrial fibrillation.
(28) The method according to (27), wherein the diastolic congestive heart failure-derived abnormality is increased ventricular stiffness or cardiac diastolic dysfunction.
(29) A method for improving cardiac diastolic dysfunction, comprising administering at least one compound selected from the group consisting of an ω3 polyunsaturated fatty acid, a pharmaceutically acceptable salt thereof, and an ester thereof, wherein the method is used for a congestive heart failure patient who has a left ventricular ejection fraction larger than 40% and maintains a left ventricular systolic function.
(30) The method according to (29), wherein the method improves at least one index for the cardiac diastolic dysfunction selected from the group consisting of a ratio of a peak filling velocity of early diastolic transmitral flow to a peak filling velocity of atrial systolic transmitral flow (E/A ratio), a deceleration time (DT), an early diastolic mitral annular velocity (E'), and a ratio of the peak filling velocity of early diastolic transmitral flow to the early diastolic mitral annular velocity (E/E' ratio).
(31) The method according to any of (24) to (30), wherein the method is used for improvement in the prognosis of the diastolic congestive heart failure.
(32) The method according to any of (24) to (31), further comprising administering a drug selected from a renin-angiotensin system inhibitor, a diuretic, a β blocker, and a Ca channel inhibitor.
(33) The method according to any of (24) to (32), wherein the ω3 polyunsaturated fatty acid, the pharmaceutically acceptable salt thereof, or the ester thereof is at least one compound selected from the group consisting of icosapentaenoic acid, docosahexaenoic acid, and α-linolenic acid, pharmaceutically acceptable salts thereof, and esters thereof.
(34) The method according to (33), wherein the method contains icosapentaenoic acid ethyl ester as an active ingredient.
(35) The method according to (33), wherein the administration is performed at a dose of 1.2 g/day or higher in terms of the total amount of icosapentaenoic acid ethyl ester and docosahexaenoic acid ethyl ester.

### ADVANTAGEOUS EFFECTS OF INVENTION

The present invention provides means of treating diastolic congestive heart failure. The pharmaceutical composition and the treatment method of the present invention improve diastolic congestive heart failure-derived symptoms, i.e., edema, dyspnea or shortness of breath, increased ventricular stiffness, cardiac diastolic dysfunction, and/or atrial fibrillation. The pharmaceutical composition and the treatment method of the present invention exert excellent therapeutic effects particularly on diastolic congestive heart failure having excess epicardial fat. The present invention provides a therapeutic agent for diastolic congestive heart failure based on a novel mechanism of action of shrinking or removing excess epicardial fat. The pharmaceutical composition or the method of the present invention is highly safe with few adverse reactions and as such, is suitable for use in the treatment of diastolic congestive heart failure in elderly people.

### DESCRIPTION OF EMBODIMENTS

Hereinafter, the present invention will be described in detail.

Polyunsaturated fatty acids (PUFAs) are defined as fatty acids each having a plurality of carbon-carbon double bonds in the molecule and classified into ω3, ω6, and the like depending on the positions of the double bonds. Examples of ω3 PUFAs include α-linolenic acid, icosapentaenoic acid (EPA), and docosahexaenoic acid (DHA). In the present invention, any of polyunsaturated fatty acid derivatives that encompass, for example, pharmaceutically acceptable salts, esters, amides, phospholipids, or glycerides of polyunsaturated fatty acids as equivalents of the polyunsaturated fatty acids can be used as an active ingredient.

The ω3 polyunsaturated fatty acid used in the present invention may be any of synthetic, semisynthetic, and natural products and may be in the form of natural oil containing them. In this context, the natural product means a product extracted or semipurified by a method known in the art from natural oil containing an ω3 polyunsaturated fatty acid or its derivative or a product further highly purified from such an extracted or semipurified product. The semi-synthetic product includes polyunsaturated fatty acids produced by microbes, etc. and also includes such polyunsaturated fatty acids or natural polyunsaturated fatty acids chemically treated by esterification, transesterification, or the like. In the present invention, these ω3 PUFAs can be used alone or in combination of two or more thereof.

In the present invention, examples of the active ingredient include ω3 polyunsaturated fatty acids, specifically, EPA, DHA, and α-linolenic acid, and their pharmaceutically acceptable salts and esters. Examples of the pharmaceutically acceptable salts and esters include: inorganic bases such as sodium salt and potassium salt; organic bases such as benzylamine salt and diethylamine salt; salts with basic amino acids such as arginine salt and lysine salt; and alkyl esters such as methyl ester and ethyl ester; esters of glycerides such as mono-, di-, and tri-glycerides. Ethyl ester is preferred. Particularly, EPA ethyl ester (EPA-E) and/or DHA ethyl ester (DHA-E) are preferred.

The purity of the an ω3 polyunsaturated fatty acid, the pharmaceutically acceptable salt thereof, or the ester thereof is not particularly limited. The content of the ω3 PUFA in all fatty acids of the pharmaceutical composition is preferably 25% by weight or higher, more preferably 50% by weight or higher, even more preferably 70% by weight or higher, further preferably 85% by weight or higher, further preferably 98% by weight or higher. In a particularly preferred embodiment, the pharmaceutical composition is substantially free from fatty acid components other than an ω3 polyunsaturated fatty acid. In the case of using, for example, EPA-E and DHA-E, the compositional ratio of EPA-E/DHA-E and the total content of EPA-E and DHA-E in all fatty acids are not particularly limited. The compositional ratio of EPA-E/DHA-E is preferably 0.8 or higher, more preferably 1.0 or higher, even more preferably 1.2 or higher. High purity of EPA-E and DHA-E, for example, the total content of EPA-E and DHA-E of 40% by weight or higher in all fatty acids (and their derivatives), is preferred. The total content of EPA-E and DHA-E is more preferably 55% by weight or higher, even more preferably 84% by weight or higher, further preferably 96.5% by weight or higher. The lower content of other long-chain saturated fatty acids is more preferred.
Even among long-chain unsaturated fatty acids, ω6, particularly, arachidonic acid, is desirably contained in a lower amount. Its content is preferably less than 2% by weight, more preferably less than 1% by weight.

EPA-E and/or DHA-E used in the present invention have fewer impurities, such as saturated fatty acids or arachidonic acid, which are unfavorable for cardiovascular events, than those of fish oil or fish oil concentrates and can exert their effects or functions without the problem of excess nutrients or excessive intake of vitamin A. In addition, these ethyl ester forms have higher oxidative stability than that of fish oil or the like, which is mainly in a triglyceride form, and can yield a sufficiently stable composition by the addition of a usual antioxidant.

A soft capsule containing highly pure EPA-E (96.5% by weight or higher) (trade name: Epadel; manufactured by Mochida Pharmaceutical Co., Ltd.) is available as a therapeutic drug for arteriosclerosis obliterans (ASO) and hyperlipemia in Japan and can be used as the EPA-E of the present invention. For example, Lovaza (GlaxoSmithKline plc; a soft capsule containing approximately 46.5% by weight of EPA-E and approximately 37.5% by weight of DHA-E) commercially available as a therapeutic drug for hypertriglyceridemia in the USA may be used as a mixture of EPA-E and DHA-E.

Purified fish oil containing an ω3 polyunsaturated fatty acid as, for example, a free fatty acid or a fatty acid constituting glyceride may be used as the active ingredient of the present invention. In one embodiment, for example, monoglyceride, diglyceride, or triglyceride of an ω3 polyunsaturated fatty acid or a combination thereof is also preferred. For example, various products containing an ω3 polyunsaturated fatty acid, a salt thereof, or an ester thereof, such as Incromega F2250, F2628, E2251, F2573, TG2162, TG2779, TG2928, TG3525, and E5015 (Croda International PLC, Yorkshire, England), and EPAX6000FA, EPAX5000TG, EPAX4510TG, EPAX2050TG, EPAX7010EE, K85TG, K85EE, and K80EE (Pronova Biopharma, Lysaker, Norway), are commercially available and can be obtained for use in the present invention.

Heart failure is diagnosed through (1) the diagnosis of the presence of symptoms or signs based on cardiac disease and the detection of causative disease and (2) the evaluation of cardiac functions (systolic function and diastolic function). The systolic function is generally evaluated on the basis of a left ventricular ejection fraction (LV ejection fraction: LVEF). LVEF of 40% or lower represents a poor left ventricular systolic function. The systolic function is evaluated using transthoracic Doppler echocardiography, transesophageal Doppler echocardiography, computed tomography (CT), magnetic resonance imaging (MRI), cardiac catheterization, or the like.

By contrast, a plurality of indexes has been proposed for the evaluation of the diastolic function. For example, Doppler echocardiography, RI cardiac pool scintigraphy, or cardiac catheterization can be used.

The progression of diastolic failure can be observed on the basis of a ratio of a peak filling velocity of early diastolic transmitral flow (E) to a peak filling velocity of atrial systolic transmitral flow (A) (E/A ratio) of a transmitral flow pattern using the Doppler echocardiography and change in the pattern. The time interval from the second heart sound to the onset of the early diastolic wave (isovolumetric relaxation time: IRT) represents active relaxation performance. The time required for the peak filling velocity of early diastolic transmitral flow to reach zero (deceleration time: DT) correlates with left ventricular stiffness. Left ventricular diastolic dysfunction exhibits IRT >100 msec, an E/A ratio < 1.0, and DT >250 msec. An early diastolic mitral annular motion velocity (E'; also indicated by e') serves as an index for the exercise of dilation and contraction in the longitudinal direction of the left ventricle. In cardiac diastolic dysfunction, the left ventricle exhibits slow exercise, i.e., E' < 8 cm/s. The E/E' ratio reflects a pulmonary arterial wedge pressure regardless of the degree of systolic dysfunction and as such, can serve as an effective index for cardiac diastolic dysfunction and also as an index for the severity of heart failure. At an E/E' ratio > 15, the elevation of the mean left ventricular diastolic pressure is confirmed, also deteriorating the survival rate of the patient.

The RI cardiac pool scintigraphy involves determining indexes for diastolic performance, i.e., a peak filling rate (PFR), which represents the maximum steep rise in the rapid filling phase of the left ventricle, and a time to peak filling rate (TPFR), which represents the duration of relaxation. The cardiac catheterization involves determining a left ventricular end-diastolic pressure (LVEDP) or a pulmonary arterial wedge pressure (in place of a left atrial pressure). Since the occurrence of diastolic dysfunction causes a secondary rise in left ventricular filling pressure in order to maintain cardiac output, the elevated left ventricular end-diastolic pressure or pulmonary arterial wedge pressure indirectly shows the presence of diastolic dysfunction. The maximum rate of the first derivation of left ventricular pressure fall (peak negative: dP/dt) and the time constant of the left ventricular pressure fall (time constant: Tau or t) are used as indexes for relaxation performance. Also, left ventricular stiffness is determined as the first derivation of the diastolic pressure-volume relation (dP/dV). Normal references are a peak filling rate (PFR) of 3.13 ± 0.85/sec, a peak negative (dP/dt) of 1864 ± 390 mmHg/sec, and a time constant (Tau or t) of 33 ± 8 msec. A secondary rise in left atrial pressure or a morphological change caused by diastolic dysfunction is widely used as a noninvasive index in the current methods for evaluating the diastolic function. A higher left atrial pressure represents that diastolic dysfunction is confirmed with a higher stage of progression (Guidelines for Treatment of Chronic Heart Failure (JCS 2010), p. 5-9).

The pharmaceutical composition of the present invention is applied to a patient with diastolic congestive heart failure. The patient with diastolic congestive heart failure is generally a chronic heart failure patient who has a left ventricular ejection fraction larger than 40% and maintains left ventricular contractility. A larger left ventricular ejection fraction represents being closer to normal left ventricular contractility. The left ventricular ejection fraction is preferably 45% or larger, more preferably 50% or larger. The left ventricular ejection fraction is determined according to (EDV - EDV) / EDV by calculating an end-diastolic volume (EDV) and an end-systolic volume (EDV) from the end-diastolic minor axis diameter (Dd) and end-systolic minor axis diameter (Ds), respectively, of the left ventricle measured in echocardiographic examination (Textbook for Cardiac Ultrasound, edited by the Japanese Society of Sonographers, issued by Ishiyaku Pub, Inc., 2001). The presence or absence of diastolic dysfunction may be confirmed using any of the methods for evaluating the diastolic function. The diastolic function used in the present specification refers to a cardiac diastolic function and specifically refers to a left ventricular diastolic function. The diastolic performance or dilation is synonymous with the diastolic function. The systolic function refers to a cardiac systolic function and specifically refers to a left ventricular systolic function. The systolic performance or contractility is synonymous with the systolic function.

The pharmaceutical composition of the present invention is preferably applied particularly to a diastolic congestive heart failure patient having excess epicardial fat, among diastolic congestive heart failure patients. The presence of excess epicardial fat is considered closely related to the pathology and prognosis of diastolic congestive heart failure. Nevertheless, only a very few attempts have been made to shrink or remove such excess epicardial fat to thereby treat or improve symptoms (e.g., edema, dyspnea or shortness of breath, increased ventricular stiffness, cardiac diastolic dysfunction, and atrial fibrillation) in diastolic congestive heart failure patients or the prognosis thereof. The present inventor has found that the administration of an ω3 polyunsaturated fatty acid, a pharmaceutically acceptable salt thereof, or an ester thereof in an effective amount can shrink the excess epicardial fat or treat diastolic congestive heart failure through the suppression of biologically active substances released from the epicardial fat.

The thickness of the epicardial fat can be visualized and measured by two-dimensional echocardiography or magnetic resonance imaging. Fluchter S et al. used the magnetic resonance imaging to determine the mean thickness of epicardial fat in healthy persons to be 3.8 mm to 4.3 mm (Fluchter S et al., Obesity (Silver Spring). 2007; 15: 870). The excess epicardial fat refers to a 5 mm or thicker epicardial fat, more preferably 7 mm or thicker epicardial fat.

The therapeutic effects on diastolic congestive heart failure according to the present invention are not particularly limited and can be confirmed by, for example, improvement in diastolic congestive heart failure-derived symptoms such as edema, dyspnea or shortness of breath, increased ventricular stiffness, cardiac diastolic dysfunction, and atrial fibrillation, the reduced amount of epicardial fat, or the decreased thickness of epicardial fat. The therapeutic effects can be confirmed by improvement in at least one of these indexes. For example, the therapeutic effects on diastolic congestive heart failure can be demonstrated if improvement in at least one of the indexes E/A ratio, DT, E', and E/E' ratio for cardiac diastolic dysfunction is confirmed in the diastolic congestive heart failure patient. Among these indexes, the E/E' ratio shows correlation with the prognosis of heart failure and is thus preferred as an index. The effects can be confirmed by qualitative improvement in at least one of the indexes. Desirably, an index that can be indicated by a numeric value is improved by at least 2% or higher, preferably 5% or higher, more preferably 10% or higher, even more preferably 20% or higher compared with the value before the treatment. Alternatively, the effects of the present invention can be confirmed by the measurement of a biomarker (e.g., brain natriuretic peptide (BNP)) level used as an index for therapeutic effects on heart failure. Alternatively, the effects of the present invention can be confirmed if the pharmaceutical composition of the present invention can improve the degree of the disease at least by one stage of the NYHA classification as a result of treatment for a given period.

In one embodiment, the pharmaceutical composition of the present invention is applied to a patient affected by lifestyle-related disease. Examples of the lifestyle-related disease include hyperlipemia, diabetes mellitus, metabolic syndrome, hypertension, and obesity. The pharmaceutical composition of the present invention is preferably applied to a patient who is affected by lifestyle-related disease and has diastolic congestive heart failure.

The dose and dosing period of the ω3 polyunsaturated fatty acid used in the present invention are set to an amount and a period sufficient for exhibiting the intended action and can be increased or decreased appropriately depending on its dosage form, administration method, the number of doses per day, the degree of symptoms, body weight, age, etc.

In the case of oral administration, the pharmaceutical composition of the present invention can be administered in one to three divided doses at a dose of, for example, 0.3 to 10 g/day, preferably 0.6 to 6 g/day, more preferably 1.0 to 4 g/day, even more preferably 1.2 to 2.7 g/day, in terms of the amount of the ω3 polyunsaturated fatty acid. If necessary, the total daily dose may be administered in a single dose or in several divided doses. The dose is 1.2 g/day or higher, preferably 1.8 g/day or higher, more preferably 2.7 g/day or higher, in terms of the amount of the ω3 polyunsaturated fatty acid particularly for treating diastolic congestive heart failure by shrinking or removing excess epicardial fat. Particularly, the pharmaceutical composition administered at a dose of 1.2 g/day or higher in terms of the total amount of EPA-E and DHA-E or at a dose of 1.2 g/day or higher in terms of the amount of EPA-E is useful in the treatment of diastolic congestive heart failure. The dosing period is at least 2 weeks or longer, preferably 1 month or longer, more preferably 3 months or longer. Also, the pharmaceutical composition is preferably administered during or after a meal, more preferably, immediately after a meal (within 30 minutes after a meal). Alternatively, the pharmaceutical composition may be administered, for example, every other day or 2 to 3 days per week.

An EPA/AA ratio is often used as an index from a pharmacological or clinical standpoint. A plasma EPA/AA ratio exceeds 1.0 in 1 week of administration, which is approximately two times the value before the administration. The dose and the administration intervals can be adjusted so that the serum ω3 polyunsaturated fatty acid concentration of the first week of the administration can be maintained as an index for continuous administration and/or the plasma EPA/AA ratio is 1.0 or larger.

The active ingredient can be administered alone as the pharmaceutical composition of the present invention or can be prepared into an appropriate pharmaceutical preparation by appropriately selecting and combining suitable excipients generally used, such as carriers or vehicles, diluents, binding agents, lubricants, colorants, flavors, if necessary sterilized water or plant oil, and further, harmless organic solvents or harmless solubilizers (e.g., glycerin and propylene glycol), emulsifiers, suspending agents (e.g., Tween 80 and gum arabic solutions), tonicity agents, pH adjusters, stabilizers, soothing agents, corrigents, aromatics, preservatives, antioxidants, buffering agents, and colorants. The preparation may contain, for example, lactose, partly pregelatinized starch, hydroxypropylcellulose, Macrogol, tocopherol, hydrogenated oil, sucrose fatty acid ester, hydroxypropylmethylcellulose, titanium oxide, talc, dimethylpolysiloxane, silicon dioxide, and carnauba wax as such excipients.

Particularly, since the ω3 polyunsaturated fatty acid is highly unsaturated, the preparation desirably contains an effective amount of at least one antioxidant selected from, for example, butylated hydroxytoluene, butylated hydroxyanisole, propyl gallate, gallic acid, pharmaceutically acceptable quinone, and α-tocopherol.

The dosage form of the preparation differs depending on the combined use form of the active ingredient of the present invention and is not particularly limited. The preparation is preferably an oral preparation and may be used in the form of, for example, tablets, film-coated tablets, capsules, microcapsules, granules, fine granules, powders, oral liquid preparations, syrups, jellies, or inhalants. Particularly, the active ingredient is preferably encapsulated in the shells of capsules, for example, soft capsules or microcapsules or orally administered in the form of tablets or film-coated tablets. Alternatively, an enteric-coated preparation or a sustained-release preparation may be orally administered. Also preferably, jellies are orally administered to dialyzed patients, patients unable to swallow, or the like.

The pharmaceutical composition of the present invention can be used in combination with a second drug other than the ω3 polyunsaturated fatty acid. The second drug may be contained in the pharmaceutical composition of the present invention or may be administered as another preparation simultaneously with the pharmaceutical composition of the present invention or separately from the pharmaceutical composition of the present invention with a time interval. Examples of the second drug include, but not particularly limited to, hypotensive drugs (e.g., renin-angiotensin system inhibitors, sympathetic β receptor blockers (β blockers), Ca channel inhibitors, α/β blockers, central α2 agonists or other centrally acting drugs, and vasodilators), nitric acid medicines, diuretics, anti-arrhythmic drugs, drugs for hyperlipemia, antithrombotic drugs, therapeutic drugs for diabetes mellitus or diabetic complications, and antiobesity drugs and preferably include renin-angiotensin system inhibitors, diuretics, sympathetic β receptor blockers (β blockers), and Ca channel inhibitors. Examples of the renin-angiotensin system inhibitors include angiotensin-converting enzyme inhibitors and angiotensin II receptor antagonists (ARB).

In one embodiment, the pharmaceutical composition of the present invention is used for improvement in the prognosis of diastolic congestive heart failure. The prognosis includes survival durations, survival rates, and hospitalization for cardiovascular reasons. The pharmaceutical composition of the present invention is particularly suitable for, for example, patients who have diastolic congestive heart failure and are difficult to completely cure by therapy.

The pharmaceutical composition of the present invention can contain pharmaceutically acceptable diluents in addition to the active ingredient. The pharmaceutical composition of the present invention may appropriately contain an antioxidant, a coating agent, a gelling agent, a corrigent, an aromatic, a preservative, an antioxidant, an emulsifier, a pH adjuster, a buffering agent, a colorant, and the like known in the art.

The pharmaceutical composition of the present invention may be formulated according to a routine method. Powders of ω3 polyunsaturated fatty acid are obtained by a method known in the art which involves, for example, drying an oil-in-water emulsion containing (A) EPA-E, (B) dietary fiber, (C) starch hydrolysates and/or low-glycemic reduced starch decomposition products, and (D) a water-soluble antioxidant under high vacuum, followed by pulverization (Japanese Patent Laid-Open No. 10-99046). The obtained EPA-E powders can be used to obtain granules, fine granules, powders, tablets, film-coated tablets, chewable tablets, sustained-release tablets, orally disintegrating tablets (OD tablets), or the like according to a routine method. The chewable tablets can be obtained by a method known in the art which involves, for example, emulsifying EPA-E in a solution of a water-soluble polymer such as hydroxypropylmethylcellulose and spraying the obtained emulsion onto an excipient such as lactose to obtain granular solids (Japanese Patent Laid-Open No. 8-157362), followed by tableting. The orally disintegrating tablets can be produced according to a method known in the art, for example, the method of Japanese Patent Laid-Open No. 8-333243, while the oral film preparation can be produced according to a method known in the art, for example, the method of Japanese Patent Laid-Open No. 2005-21124.

Desirably, the pharmaceutical composition of the present invention releases the active ingredient, which is then absorbed so that the active ingredient can exert its pharmacological effects. Desirably, the combination drug of the present invention has at least any one or more effects of a preparation that is excellent in the release of the active ingredient, excellent in the absorbability of the active ingredient, excellent in the dispersibility of the active ingredient, excellent in the storage stability of the combination drug, convenient for intake by a patient, or excellent in compliance.

### EXAMPLES

Next, the present invention will be described specifically with reference to Examples. However, the present invention is not limited by these Examples.

(Example 1) Therapeutic effect of administered EPA-E on diastolic congestive heart failure having excess epicardial fat

### (1) Treatment of diastolic congestive heart failure

EPA-E is administered at a daily dose of 1200 to 2700 mg for at least 3 months to each chronic heart failure patient who has a left ventricular ejection fraction larger than 40% and maintains a left ventricular systolic function. Change in various symptoms associated with heart failure is confirmed during the dosing period. After the completion of the dosing period, at least one of heart failure-associated indexes, i.e., edema, dyspnea or shortness of breath, increased ventricular stiffness, cardiac diastolic dysfunction, and atrial fibrillation is alleviated or suppressed.

### (2) Treatment of diastolic congestive heart failure having excess epicardial fat

EPA-E is administered at a daily dose of 1800 mg for 3 months to each diastolic congestive heart failure patient confirmed in advance to have 5 mm or thicker excess epicardial fat by echocardiographic examination. Change in various symptoms associated with heart failure is confirmed during the dosing period. After 3 months, the state of epicardial fat is confirmed again. The amount or thickness of epicardial fat is reduced, while at least one of heart failure-associated indexes, i.e., edema, dyspnea or shortness of breath, increased ventricular stiffness, cardiac diastolic dysfunction, atrial fibrillation, and the amount or thickness of epicardial fat is alleviated or suppressed.

### (Example 2) Therapeutic effect of administered EPA-E on diastolic congestive heart failure

Diastolic congestive heart failure patients were targeted and divided into an EPA-E-administered group and an EPA-E-non-administered group. The EPA-E-administered group received the oral administration of EPA-E (trade name: Epadel; manufactured by Mochida Pharmaceutical Co., Ltd.) at a daily dose of 1800 mg for 6 months. The EPA-E-non-administered group received neither EPA nor its derivative during the test period. Another hypotensive or antilipidemic agent was not administered to any of these groups during the test period. The diastolic function of each patient was evaluated by echocardiography before the start of the test and after the completion of the test (6 months later). The evaluation items used were 1) a ratio of a peak filling velocity of early diastolic transmitral flow (a peak filling velocity of mitral inflow during early diastole: E) to a peak filling velocity of atrial systolic transmitral flow (a peak filling velocity of mitral inflow during atrial contraction: A) (E/A ratio), 2) the time required for the peak filling velocity of mitral inflow during early diastole to reach zero (deceleration time: DT), 3) an early diastolic mitral annulus velocity (E'), and 4) an E/E' ratio. The degree of improvement in these indexes was confirmed for each case corresponding to 1) an E/A ratio < 1.0, 2) DT > 250 msec, 3) E' < 8 cm/s, or 4) an E/E' ratio > 15 as an index for cardiac diastolic dysfunction. The results are shown in Table 1. The rate of change was determined as an average of % change from the value (defined as 100) before the start to the value after 6 months in each case.

[Table 1]

**Table 1**

| Evaluation item | Target case | EPA-E-non-administered group | | | EPA-E-administered group | | |
|---|---|---|---|---|---|---|---|
| | | Mean before start (the number of cases) | Mean after 6 months (the number of cases) | Rate of change | Mean before start (the number of cases) | Mean after 6 months (the number of cases) | Rate of change |
| E/A ratio | E/A ratio<1.0 | 0.651 (6) | 0.640 (6) | 0.4% reduction (deterioration) | 0.669 (14) | 0.692 (14) | 3.7% rise (improvement) |
| DT | DT>250 msec | 284.0 (3) | 263.7 (3) | 7.1 % reduction (improvement) | 271.3 (6) | 241.7 (6) | 10.9% reduction (improvement) |
| E' | E'>8 cm/s | 5.343 (7) | 5.157 (7) | 3.5% reduction (deterioration) | 4.483 (18) | 4.539 (18) | 2.3% rise (improvement) |
| E/E' ratio | E/E' ratio>15 | 18.36 (2) | 18.95 (2) | 3.2% rise (deterioration) | 18.09 (4) | 13.14 (4) | 27.4% reduction (improvement) |

These results demonstrated improvement in cardiac diastolic dysfunction in the EPA-E-administered group.

## Claims

1. A pharmaceutical composition for the treatment of diastolic congestive heart failure containing at least one active ingredient selected from the group consisting of an ω3 polyunsaturated fatty acid, a pharmaceutically acceptable salt thereof, and an ester thereof.

2. The pharmaceutical composition according to claim 1, wherein the diastolic congestive heart failure is with the deposition of 5 mm or thicker epicardial fat.

3. The pharmaceutical composition according to claim 1, wherein the diastolic congestive heart failure is with the deposition of 7 mm or thicker epicardial fat.

4. The pharmaceutical composition according to any one of claims 1] to 3, wherein the pharmaceutical composition is used for improvement in any one or more diastolic congestive heart failure-derived abnormalities (abnormalities) selected from edema, dyspnea or shortness of breath, increased ventricular stiffness, cardiac diastolic dysfunction, and atrial fibrillation.

5. The pharmaceutical composition according to claim 4, wherein the diastolic congestive heart failure-derived abnormality is increased ventricular stiffness or cardiac diastolic dysfunction.

6. The pharmaceutical composition according to any one of claims 1] to 5, wherein the pharmaceutical composition is used for improvement in the prognosis of the diastolic congestive heart failure.

7. The pharmaceutical composition according to any one of claims 1] to 6, wherein the pharmaceutical composition is used in combination with a drug selected from a renin-angiotensin system inhibitor, a diuretic, a β blocker, and a Ca channel inhibitor.

8. The pharmaceutical composition according to any one of claims 1] to 7, wherein the ω3 polyunsaturated fatty acid, the pharmaceutically acceptable salt thereof, or the ester thereof is at least one compound selected from the group consisting of icosapentaenoic acid, docosahexaenoic acid, and α-linolenic acid, pharmaceutically acceptable salts thereof, and esters thereof.

9. The pharmaceutical composition according to claim 8, wherein the pharmaceutical composition contains icosapentaenoic acid ethyl ester as an active ingredient.

10. The pharmaceutical composition according to claim 8, wherein the pharmaceutical composition is administered, for use, at a dose of 1.2 g/day or higher in terms of the total amount of icosapentaenoic acid ethyl ester and docosahexaenoic acid ethyl ester.
